Europäisches Patentamt

European Patent Office

Office européen des brevets

(19)

(11) Veröffentlichungsnummer : **0 022 138
B1**

## (12) EUROPÄISCHE PATENTSCHRIFT

(45) Veröffentlichungstag der Patentschrift :
30.03.83

(21) Anmeldenummer : 79200378.2

(22) Anmeldetag : 09.07.79

(51) Int. Cl.³ : **C 12 M   1/08**

(54) **Schlaufenreaktor zum Begasen von Flüssigkeiten.**

(43) Veröffentlichungstag der Anmeldung :
**14.01.81 Patentblatt 81/02**

(45) Bekanntmachung des Hinweises auf die Patenterteilung : **30.03.83 Patentblatt 83/13**

(84) Benannte Vertragsstaaten :
**AT CH DE FR IT**

(56) Entgegenhaltungen :
**BE A 471 486
DE C 500 703
FR A 469 300
FR A 569 304
FR A 1 578 295
FR A 2 229 451
FR A 2 357 488
FR A 2 406 664
US A 1 727 601**

(73) Patentinhaber : **Chemap AG
Alte Landstrasse 415
CH-8708 Männedorf (CH)**

(72) Erfinder : **Müller, Hans, Dr. Ing.
Im Allmendli
CH-8703 Erlenbach (CH)**

(74) Vertreter : **Herrmann, Peter
Chemap AG Alte Landstrasse 415
CH-8708 Männedorf (CH)**

Schlaufenreaktor zum Begasen von Flüssigkeiten

Gegenstand der Erfindung ist ein Schlaufenreaktor zum Begasen von Flüssigkeiten, insbesondere zur aeroben Züchtung von Mikroorganismen.

Schlaufenreaktoren sind Blasensäulen, welche durch den Einbau eines koaxialen Leitrohres der mit Gas beladenen aufsteigenden Flüssigkeit einen definierten Umlauf vorschreiben. Man unterscheidet mehrere Arten von Schlaufenreaktoren. Im einfachsten Fall wird Gas durch eine zentrale Düse am Boden des Reaktors in das Leitrohr eingeführt. Die Gasblasen nehmen die Flüssigkeit nach dem Prinzip der Mammutpumpe nach oben mit. Die Flüssigkeit strömt unter Entgasung über den oberen Rand zwischen Reaktormantel und Leitrohr nach unten zurück. Durch einen zusätzlichen Treibstrahl kann die Gasdispergierung wesentlich verbessert werden.

Schlaufenreaktoren werden bei vielen technischen Gas-Flüssigkeitsreaktionen eingesetzt. So beschreibt die DE-C-1 205 514 die Durchführung des Oxoverfahrens in einem Schlaufenreaktor, und die DE-A-25 06 934 beschreibt einen Schlaufenreaktor für Bioreaktionen. Trotz der geringen Blasengrösse im Bereich von 10-1000 Micrometer stellen die bekannten Schlaufenreaktoren bei Reaktionen, beispielsweise bei Bioreaktion zur aeroben Züchtung von Mikroorganismen, eine nicht ausreichende Versorgung dieser Mikroorganismen mit Sauerstoff dar.

Aufgabe der Erfindung ist es daher, einen Schlaufenreaktor zu schaffen, der eine bessere Ausnutzung des zur Begasung verwendeten Gases ermöglicht.

Diese Aufgabe wird erfindungsgemäss nach Patentanspruch 1 gelöst und ist dadurch gekennzeichnet, dass das Leitrohr mit scharfkantigen Spänen ausgefüllt ist.

Die Erfindung soll anhand von Zeichnungen beispielsweise näher beschrieben werden.

Es zeigen :

Figur 1 den erfindungsgemässen Schlaufenreaktor im Längsschnitt,

Figur 2 eine Variante des Schlaufenreaktors gemäss Figur 1 mit unterteiltem Leitrohr.

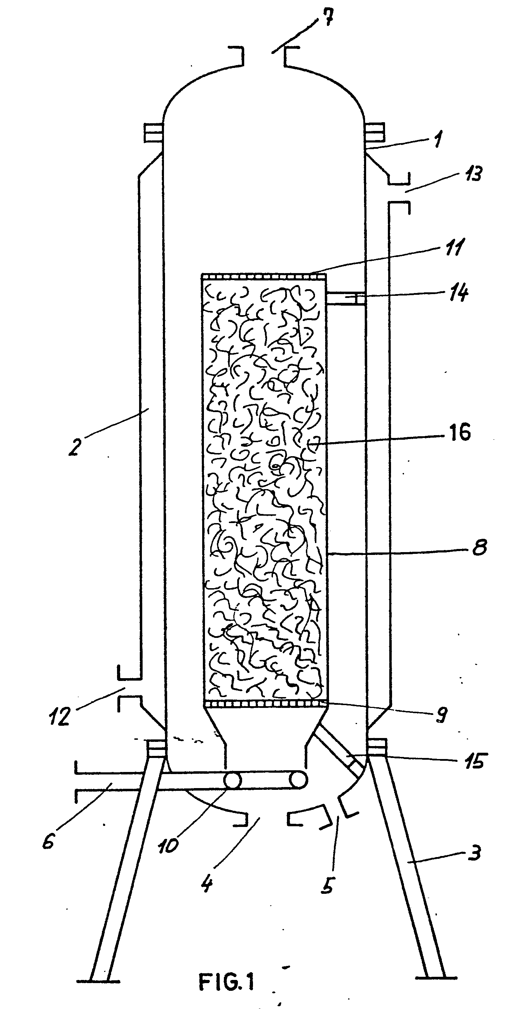

In Figur 1 besteht der Reaktor aus einem zylindrischen Kessel 1 mit einem Doppelmantel 2. Der Kessel 1 ist auf Stützen 3 aufgestellt und besitzt einen Eintrittstutzen 4 für das zu begasende Medium und einen Austrittstutzen 5 für das Reaktionsprodukt. Ein weiterer Stutzen 6 ist für die Zuluft und ein Stutzen 7 im Kopf des Reaktors für die Abluft vorgesehen. Im Innern des Behälters befindet sich das koaxial angebrachte Leitrohr 8 mit einer Lochplatte 9, die den gesamten Querschnitt ausfüllt. Ein Belüftungsringrohr 10 weist mit seinen Gasaustrittsöffnungen in den Leitrohrquerschnitt hinein. Ein gleiches Lochblech 11 verschliesst das Leitrohr 8 gegen oben. Das Innere des Leitrohres 8 ist vollständig mit Spänen 16 ausgefüllt. Es haben sich vorzugsweise Metall- und Kunststoffspäne als geeignet erwiesen, abhängig von der Art des zu begasenden Mediums. Bei der Begasung von Medien zur Züchtung von Mikroorganismen haben sich vorzugsweise Späne aus Edelstahl als geeignet erwiesen. Es wird drehspanartigen Metallspänen mit scharfen Kanten aus rostfreiem Stahl den Vorzug gegeben. Der Kühlmantel ist mit einem Eintritt 12 und einem Austritt 13 versehen. Das Leitrohr 8 ist in bekannter Weise durch Verstrebungen 14 und 15 befestigt.

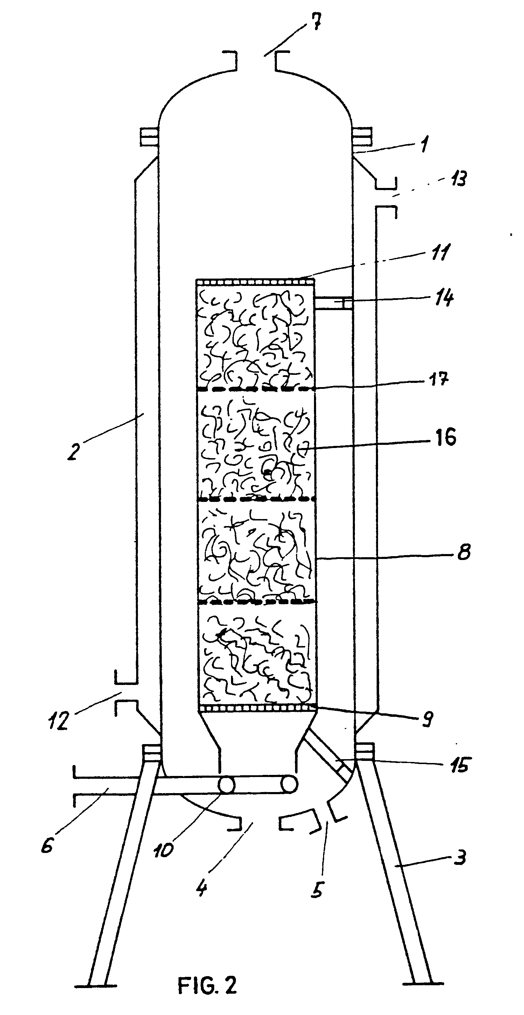

Figur 2 unterscheidet sich von Figur 1 lediglich dadurch, dass das Leitrohr 8 durch horizontale Einbauten 17 in zwei oder mehrere Abteilungen unterteilt wird. Diese Einbauten bestehen vorzugsweise aus Drahtsieben oder perforierten Metallscheiben. Die Öffnungen können rund oder eckig gestaltet sein.

Im Betrieb wird der Reaktor, der beispielsweise mit Mikroorganismen und einem handelsüblichen Nährsubstrat versehen ist, durch gereinigte und wenn erforderlich sterile Druckluft über die Zuluftleitung 6 und die Ringleitung 10 begast. Dabei steigen Gas und flüssiges Medium durch die Mammutpumpenwirkung nach oben. An den scharfen Kanten der Späne werden koaleszierende Gasblasen bei genügend hoher Strömungsgeschwindigkeit immer wieder zerteilt und somit deren Oberfläche vergrössert. Das Lochblech 11 im oberen Teil des Leitrohres verhindert ein Austreten der Späne und lässt nur das begaste Medium hindurchtreten. Im Dom des Behälters trennt sich überschüssiges Gas und verlässt als Abgas über den Stutzen 7 den Kessel 1. Die flüssige Phase hingegen fällt durch die Schwerkraft über den oberen Rand des Leitrohres zwischen letzterem und der Kesselwand hinab und wird erneut durch das über den Ring 10 austretende Gas durch das Leitrohr 8 gefördert. Bei einer kontinuierlichen Arbeitsweise wird ständig ein bestimmter Teil der begasten Lösung über den Stutzen 5 aus dem Kessel 1 abgezogen, während neues unbegastes Medium über den Eintritt 4 nachgeführt werden kann.

Die Unterteilung des Leitrohres in mehrere Abteilungen ist für Behälter grosser Höhe von besonderem Vorteil, da ohne sie der Druck der Späne auf die unteren Schichten so gross würde, dass die Späne zusammengedrückt und somit dem Luftdurchtritt einen zu hohen Widerstand entgegensetzen würden. Bei der Abtrennung in einzelne Abteilungen ist es wichtig, dass die Einbauten so grosse Durchlässe aufweisen, dass sowohl der Gas- als auch der Flüssigkeitsdurchtritt weitgehend ungehindert erfolgen kann.

Die beschriebene Begasungsvorrichtung ist hervorragend zur Begasung von Flüssigkeiten mit Luft, insbesondere zur Begasung von Mikroorganismen geeignet.

Als Masszahl für die Wirksamkeit einer Begasung kann der in der mikrobiologischen Technik verwendete Sauerstoffeintrag OTR (Oxygen Transfer Rate) angesehen werden. Unter dem OTR versteht man die mMol Sauerstoff, die pro Liter Medium in einer Stunde bei 1 atm in das flüssige Medium eingetragen werden.

Um Vergleichswerte mit und ohne Späne im Leitrohr zu erhalten, wurden die OTR-Werte bestimmt. Die Messung wurde nach einer modifizierten Methode nach C.M. Cooper et al., Ind. Eng. Chem. *36*, 504 (1944) durchgeführt. Diese Methode beruht darauf, dass eine Natriumsulfitlösung unter Zusatz eines Katalysators mit einer definierten Luftmenge unter bestimmten Bedingungen belüftet wird. Die Geschwindigkeit der Oxidation wird in bekannter Weise in OTR umgerechnet.

Bei einer Belüftungsrate von 2 vvm (Volumen Gas das pro Volumen Flüssigkeit pro Minute einzubringen ist) in 800 l Wasser wurden unter Verwendung von Kobaltsulfat ($CoSO_4$) als Katalysator folgende OTR-Werte erhalten :

ohne Späne
240 mMol/l · h · atm
mit Späne
490 mMol/l · h · atm

Diese Ergebnisse zeigen, dass die Belüftung mit der erfindungsgemässen Vorrichtung doppelt so wirksam ist wie mit Schlaufenreaktoren ohne Füllung des Leitrohres.

### Ansprüche

1. Schlaufenreaktor zum Begasen von Flüssigkeiten, insbesondere zur aeroben Züchtung von Mikroorganismen, der ein koaxial angebrachtes, mit einer Gaszuführung versehenes Leitrohr aufweist, dadurch gekennzeichnet, dass das Leitrohr (8) mit scharfkantigen Spänen (16) ausgefüllt ist.

2. Vorrichtung nach Anspruch 1, dadurch gekennzeichnet, dass die Späne (16) aus Metall hergestellt sind.

3. Vorrichtung nach Anspruch 1, dadurch gekennzeichnet, dass die Späne (16) aus Kunststoff hergestellt sind.

4. Vorrichtung nach einem der Ansprüche 1 bis 3, dadurch gekennzeichnet, dass innerhalb des Leitrohres (8) scheibenförmige gas- und flüssigkeitsdurchlässige Einbauten (17) zur Aufteilung in zwei oder mehrere Abteilungen angebracht sind.

5. Vorrichtung nach Anspruch 4, dadurch gekennzeichnet, dass die Einbauten (17) aus Sieben bestehen.

6. Vorrichtung nach Anspruch 4, dadurch gekennzeichnet, dass die Einbauten (17) aus perforierten Platten bestehen.

7. Vorrichtung nach Anspruch 1, dadurch gekennzeichnet, dass das Leitrohr mit einer perforierten Bodenplatte (9) und einer perforierten Deckplatte (11) verschlossen ist.

8. Vorrichtung nach Anspruch 7, dadurch gekennzeichnet, dass der Zwischenraum zwischen Boden- und Deckplatte (9, 11) vollständig mit Spänen (16) ausgefüllt ist.

### Claims

1. Loop-tye reactor for aeration of liquids, particularly for aerobic cultivation of microorganisms equipped with a coaxially fixed draft-tube (8) supplied with a gas-admission-pipe (10), wherein the draft tube (8) is filled up with sharp-edged turnings (16).

2. An arrangement according to claim 1, wherein the turnings (16) are made of metal.

3. An arrangement according to claim 1, wherein the turnings (16) are made of plastics.

4. An arrangement according to one of the preceding claims, wherein the inside of the draft-tube (8) is supplied with disc-shaped baffles (17) permeable to gas and liquids for separation in two or more compartments.

5. An arrangement according to claim 4, wherein the baffles (17) are made of sieves.

6. An arrangement according to claim 4, wherein the baffles are made of perforated plates.

7. An arrangement according to claim 1, wherein the draft-tube (8) is closed with a perforated bottomplate (9) and with a perforated cover-plate (11).

8. An arrangement according to claim 7, wherein the space between the bottom- and cover-plate (9, 11) is completely filled up with turnings.

### Revendications

1. Réacteur à boucle pour l'introduction de gaz dans des liquides, en particulier pour la culture aérobie de microorganismes, équipé d'une conduite (8) coaxialement disposée ; caractérisé en ce que la conduite (8) pourvue d'une amenée de gaz (10), est remplie de copeaux (16) à arêtes vives.

2. Appareil selon la revendication 1, caractérisé en ce que les copeaux (16) sont en métal.

3. Appareil selon la revendication 1, caractérisé en ce que les copeaux (16) sont en matière synthétique.

4. Appareil selon une des revendications 1 à 3, caractérisé en ce que, à l'intérieur de la conduite (8), sont disposés des éléments (17) en forme de disques perméables aux gaz et aux liquides ; éléments assurant la division de la conduite (8) en deux ou plusieurs compartiments.

5. Appareil selon la revendication 4, caractérisé en ce que les éléments (17) consistent en des tamis.

6. Appareil selon la revendication 4, caractérisé en ce que les éléments (17) consistent en des plaques perforées.

7. Appareil selon la revendication 1, caractérisé en ce que la conduite (8) est fermée d'une plaque-fond (9) perforée et d'une plaque de couverture (11) perforée.

8. Appareil selon la revendication 7, caractérisé en ce que le volume compris entre la plaque-fond (9) et la plaque de couverture (11) est complètement remplie de copeaux (16).

FIG.1

FIG. 2